(19) Europäisches Patentamt / European Patent Office / Office européen des brevets

(11) **EP 3 582 840 B1**

(12) **EUROPÄISCHE PATENTSCHRIFT**

(45) Veröffentlichungstag und Bekanntmachung des Hinweises auf die Patenterteilung:
**15.09.2021   Patentblatt 2021/37**

(51) Int Cl.:
**F04B 53/00** (2006.01)     **A61M 16/00** (2006.01)
**A61B 5/087** (2006.01)     **G01F 1/46** (2006.01)

(21) Anmeldenummer: **18709915.5**

(22) Anmeldetag: **16.02.2018**

(86) Internationale Anmeldenummer:
**PCT/DE2018/000042**

(87) Internationale Veröffentlichungsnummer:
**WO 2018/149435 (23.08.2018 Gazette 2018/34)**

(54) **VORRICHTUNG ZUR BEATMUNG MIT DIFFERENZDRUCKSENSOR**

BREATHING APPARATUS COMPRISING A DIFFERENTIAL PRESSURE SENSOR

DISPOSITIF D'ASSISTANCE RESPIRATOIRE À CAPTEUR DE PRESSION DIFFÉRENTIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.02.2017   DE 102017001558**

(43) Veröffentlichungstag der Anmeldung:
**25.12.2019   Patentblatt 2019/52**

(73) Patentinhaber: **Weinmann Emergency Medical Technology GmbH + Co. KG**
**22525 Hamburg (DE)**

(72) Erfinder:
• **DIEHL, Marcus**
  **22587 Hamburg (DE)**
• **HEIN, Stefan**
  **22529 Hamburg (DE)**
• **PULLA, Matthias**
  **22459 Hamburg (DE)**
• **HERRMANN, Frank**
  **25355 Barmstedt (DE)**

(74) Vertreter: **Klickow & Wetzel PartGmbB**
**Jessenstraße 4**
**22767 Hamburg (DE)**

(56) Entgegenhaltungen:
EP-A1- 0 331 772        EP-A1- 0 552 916
DE-A1- 10 035 938      DE-A1-102014 119 146
US-A- 4 403 514         US-A- 5 379 650
US-A1- 2008 092 898

• KIRKNESS J P ET AL: "Pitot-tube flowmeter for quantification of airflow during sleep;Quantitative flowmeter for polysomnography", PHYSIOLOGICAL MEASUREMENT, INSTITUTE OF PHYSICS PUBLISHING, BRISTOL, GB, Bd. 32, Nr. 2, 22. Dezember 2010 (2010-12-22), Seiten 223-237, XP020204344, ISSN: 0967-3334, DOI: 10.1088/0967-3334/32/2/006

**Beschreibung**

Die Erfindung betrifft eine Vorrichtung zur Beatmung zur Messung des Differenzdrucks in einer Vorrichtung zur Beatmung. Insbesondere ist an eine Anwendung bei mobilen Notfallbeatmungsgeräten gedacht.

Im Falle unzureichender oder ausbleibender eigenständiger Atmung eines sauerstoffbedürftigen Lebewesens, muss die Atemarbeit als lebenserhaltende Maßnahme von einem Dritten entweder manuell, beispielsweise durch Mund-zu-Mund-Beatmung oder mithilfe von Beatmungsbeuteln, oder maschinell mit einem Beatmungsgerät durchgeführt werden, um den Gasaustausch in der Lunge sicherzustellen und so die Organe mit Sauerstoff zu versorgen sowie CO2 abzuatmen.

Eine sichere Methode ist nach dem Stand der Technik die maschinelle Beatmung, die in Form von Beatmungsgeräten zur medizintechnischen Ausstattung von Rettungskräften und Klinikpersonal gehört.

Moderne Beatmungsgeräte und -verfahren bieten mit einer Volumen- und/oder Druckkontrolle wichtige Funktionen zu einer effektiven und gleichzeitig schonenden Beatmung von Patienten. Ein zu hoher Druck kann das Lungengewebe schädigen, während ein zu kleines Beatmungsvolumen eine Unterversorgung mit Sauerstoff zur Folge hat. Auch die Frequenz der Beatmung ist mit bekannten Beatmungsgeräten präzise an internationale Richtlinien anpassbar, die eine effektive Beatmung, auch im Falle von Wiederbelebungsmaßnahmen, gewährleisten sollen.

Für eine zuverlässige und genaue Regelung des Volumenstromes und des Druckes der Beatmung mit einem Beatmungsgerät müssen diese Parameter in ihrem gesamten Dynamikbereich hinreichend genau bestimmt werden. In Bezug auf die Bestimmung eines vorliegenden Volumenstromes ergibt sich bei einer herkömmlichen Messanordnung eines Differenzdruck-Messverfahrens im Sinne einer einfachen Blende oder eines einfachen Widerstandes eine quadratische Abhängigkeit, die im Bereich geringer Volumenströme nur geringe Änderungen des messbaren Differenzdruckes erzeugt und damit eine schlechtere Auflösbarkeit als bei größeren Volumenströmen bedeutet.

Die Bestimmung des Volumenstromes des Atemgases in einer Atemgasleitung erfolgt in Beatmungsgeräten in der Regel mithilfe einer Differenzdruck-Messvorrichtung, die zumindest eine Differenzdruck-Messstrecke und einen Differenzdrucksensor aufweist. Die Differenzdruck-Messstrecke erzeugt abhängig vom Volumenstrom des durch die Differenzdruck-Messstrecke strömenden Atemgases an den Messausgängen einen Differenzdruck, der mittels des Differenzdrucksensors erfasst und ggf. digitalisiert wird. Die Volumenstrom-Differenzdruck-Kennlinie der Differenzdruck-Messvorrichtung setzt sich somit zumindest aus der Volumenstrom-Differenzdruck-Kennlinie der Differenzdruck-Messstrecke und der Kennlinie des Differenzdrucksensors zusammen.

Aus dem Stand der Technik sind verschiedene Sensoreinrichtungen zur Messung des Volumenstromes mithilfe der Erfassung eines Differenzdrucks bekannt.

Das Dokument "Pitot-tube flowmeter for quantification of airflow during sleep" der Autoren J P Kirkness et al. offenbart die Verwendung einer Pitot-Rohr-Volumenstrommesseinrichtung zur Verwendung in der Polysomnographie. Die Lehre basiert auf der Messung des Drucks in der Mitte des Gasstromes entgegen der Messung des Drucks am Rand des Gasstromes in anderen Messeinrichtungen, wobei im Vergleich zu anderen differenzdruckbasierten Volumenstrommesseinrichtungen wie dem Hans Rudolph- und dem Fleisch-Pneumotachograph sehr geringe Druckänderungen bei variablem Volumenstrom realisiert ist.

Das Dokument EP 0 552 916 A1 offenbart die Verwendung eines Seitenarm-Strömungsmessers für Beatmungsgeräte. In einer ersten Ausführungsform ist in einem linear verlaufenden Strömungskanal in einem ersten Bereich mit einem ersten Durchmesser am Rand des Strömungskanals eine erste Öffnung und in Strömungsrichtung hinter der ersten Öffnung in einem zweiten Bereich mit einem geringeren Durchmesser als der erste Bereich eine zweite Öffnung am Rand des Strömungskanals angeordnet. An die Öffnungen sind Leitungen angeschlossen, die zu einem Seitenarm miteinander verbunden sind. In diesem Seitenarm wird der Volumenstrom eines durch den Seitenarm strömenden Gases mittels einer Volumenstrommesseinrichtung bestimmt.

In einer weiteren offenbarten Anordnung ist mit einem Strömungskanal konstanten Durchmessers an zwei in Strömungsrichtung hintereinander am Rand der Strömungskanals angeordneten Öffnungen ein Seitenarm verbunden, in dem zwei Venturi-Element im Sinne eines Bereichs mit verringertem Durchmesser angeordnet sind. Zwischen den Venturi-Elementen ist ein Microbridge-Flusssensor angeordnet. Mit dieser Anordnung lässt sich eine lineare Abhängigkeit des Flusses im Seitenarm und dem Fluss im Hauptarm erreichen.

Das Dokument DE10035938 offenbart ein in der Atemluftleitung eines Beatmungsgeräts angeordnetes Ventil, das auch als Strömungswiderstand für eine Differenzdruckmessung zur Bestimmung des Volumenstroms einsetzbar ist. In einer Ausführungsform werden in Strömungsrichtung geneigte an in Strömungsrichtung bei Inspiration vor und hinter dem Ventil im linear verlaufenden Strömungskanal angeordnete Messöffnungen gezeigt.

Das Dokument DE102014119146 offenbart eine Sensorvorrichtung zur Messung des Volumenstromes im Rahmen einer maschinellen Beatmung. Zur Messung des Volumenstromes weist die Sensorvorrichtung ein Widerstandelement mit einem Strömungswiderstand auf, wobei der Differenzdruck zwischen in Strömungsrichtung vor und hinter dem Widerstandelement mit einem zylinderförmigen Strömungskanal verbundenen Messleitungen gemessen wird.

Das Dokument EP0331772 zeigt einen Differenzdruckmesser für bidirektionale Gasströme insbesondere für die Beatmungsüberwachung. Zu Linearisierung des Differenzdrucks wird ein speziell gestalteter Strömungswiderstand in einem Strömungskanal angeordnet. Zur Verbesserung der Messeigenschaften wird vorgeschlagen, den Differenzdrucksensor unmittelbar an den Gasströmungswiderstand anzuschließen, sodass ein nachteiliger Einfluss auf den Frequenzgang des Drucksensorsignals, insbesondere bei höheren Luftdurchsätzen, vermieden wird.

Das Dokument US5379650 offenbart eine Vorrichtung zur Messung des Volumenstromes mittels einer Differenzdruckmessung insbesondere für Beatmungsgeräte. Die Messöffnungen sind in einem Einsatz im Strömungskanal derart angeordnet, dass die Öffnungen etwa in der Mitte des Strömungskanals liegen. Zwischen den Messöffnungen wird der Durchmesser des Strömungskanals verringert. Durch die Form des Einsatzes im Strömungskanal ist der Dynamikbereich des Sensors modifizierbar.

Das Dokument US4403514 offenbart eine Modifikation der Differenzdruckmessstrecke eines Pneumatachographs unter der Verwendung von Pitot-Rohrartig an einen linear ausgebildeten Strömungskanal angeschlossenen Messleitungen, wobei der Durchmesser des Strömungskanals im Bereich des Messöffnungen vergrößert ist. Im Strömungskanal werden Umlenkplatten angeordnet, die die Strömung des Atemgases zur Verbesserung des Messverhaltens des Sensors dienen.

Das Dokument US2008092898 offenbart eine Volumenstrommessvorrichtung für Beatmungsgeräte, wobei zwei Messleitungen mit jeweils einer Messöffnung in einen zylindrischen Strömungskanal derart hineinragen, dass die Messöffnungen etwa in der Mitte des Strömungskanals angeordnet sind. Die Messöffnungen sind in Strömungsrichtung entgegengesetzt ausgerichtet und die Messleitungen sind in einer Ausführungsform entgegen der Strömungsrichtung in der Inspirationsphase geneigt. Die Neigung der Messleitungen erfolgt von einer am Gesicht eines Patienten anzubringenden Maske weg, sodass Raum für Anschlussschläuche gegeben ist und der Sensor nur eine geringe axiale Länge aufweisen muss.

Eine Aufgabe der Erfindung ist es, eine Vorrichtung zur Beatmung zu schaffen, die eine Differenzdruck-Messvorrichtung aufweist, die eine verbesserte Volumenstrom-Differenzdruck-Kennlinie zur Bestimmung des Volumenstromes in einem Strömungskanal eines Beatmungsgerätes aufweist, sodass eine bessere Auflösbarkeit im Bereich geringer Volumenströme gegeben ist.

Diese Aufgabe wird erfindungsgemäß dadurch gelöst, dass die Vorrichtung zur Beatmung eine Differenzdruck-Messvorrichtung aufweist, mit deren Differenzdruck-Messstrecke eine verbesserte Volumenstrom-Differenzdruck-Kennlinie realisierbar ist.

Eine erfindungsgemäße Vorrichtung zur Beatmung weist eine Atemgasleitung auf, durch die ein Atemgas zu einem Patienten hin leitbar ist. In die Atemgasleitung ist bereichsweise eine Druck-Messstrecke integriert, die als eine Differenzdruck-Messstrecke ausgebildet ist. In einer vorteilhaften Ausführungsform der Erfindung ist die Druck-Messstrecke in einen Adapter integriert, der in die Atemgasleitung einsetzbar ist.

In einer vorteilhaften Ausführungsform der Erfindung, weist der Adapter neben der Differenzdruck-Messstrecke zusätzlich ein Überdruckschutzventil auf, durch das ein schneller Druckausgleich für den Fall eines Überdruckes in der Atemgasleitung realisierbar ist.

Die Differenzdruck-Messstrecke weist einen Strömungskanal auf, der erfindungsgemäß im Wesentlichen ringförmig oder als ein Ringsegment ausgebildet ist und an seiner radial außenliegenden Begrenzungsfläche zwei Messöffnungen aufweist. An die Messöffnungen schließt jeweils, integriert in einen Druckmessstutzen, eine rohrartig ausgebildete Messleitung an, die jeweils zu einem Messausgang der Differenzdruck-Messstrecke führt. Weiterhin ist auch an eine hülsenartige und/oder eine zylinderhohlwandartige Realisierung des Strömungskanals gedacht.

Die Messleitungen weisen zumindest im Bereich der Messöffnungen eine wesentliche, tangential zu der äußeren Begrenzungsfläche des Strömungskanals gerichtete Komponente auf. Weiterhin weist der Strömungskanal der Differenzdruck-Messstrecke in einer vorteilhaften Ausführungsform der Erfindung einen geringeren Querschnitt auf, als die anschließende Atemgasleitung.

Mit den Messausgängen verbindbar ist eine Differenzdruckmesseinheit, die mithilfe mindestens eines Differenzdrucksensors oder mindestens zwei einfacher Drucksensoren und weiterer elektronischer Komponenten, wie beispielsweise einem Analog-Digital-Wandler, die analogen Druckwerte in digitale Messwerte wandelt und auswertbar macht. Ebenfalls ist erfindungsgemäß an die Verwendung einer Differenzdruckmesseinheit, realisiert als ein Differenzdrucksensor mit einer digitalen Schnittstelle zur Ausgabe der Druckmesswerte, gedacht.

Die Messöffnungen sind im Strömungskanal der Differenzdruck-Messstrecke derart angeordnet, dass bei einer gegebenen Strömungsrichtung an dem ersten, in Strömungsrichtung vor dem zweiten Messausgang angeordneten Messausgang, aufgrund der zwischen den Messöffnungen auftretenden Wandreibung zwischen Atemgas und Strömungskanal, ein geringerer statischer Druck abfällt, als bei dem zweiten, in Strömungsrichtung hinter dem ersten Messausgang angeordneten Messausgang. Dadurch, dass die Messleitungen im Bereich der Messöffnungen eine wesentliche tangentiale Komponente aufweisen, ist der Querschnitt der Messöffnungen im Vergleich zum Querschnitt der Messleitungen in der Oberfläche des Strömungskanals vergrößert.

Erfindungsgemäß liegt die Ausrichtung der Messleitungen zumindest im Bereich der Messöffnung am Strömungskanal in einem Winkelbereich von 0° bis 60° gemessen von der durch die lokale Tangente definierten Achse in Richtung einer dazu orthogonalen, sich in radialer Richtung erstreckenden Achse. Ein Winkel von 0° entspricht dabei einer vollständig tangentialen Ausrichtung und ein Winkel von 90° einer vollständig radialen Ausrichtung der Messleitung in Bezug auf die radial außenliegende Begrenzungsfläche des ringförmig- oder ringsegmentförmig ausgebildeten Strömungskanals. In einer besonders vorteilhaften Ausführungsform der Erfindung liegt die Ausrichtung der Messleitung zumindest im Bereich der Messöffnung am Strömungskanal in einem Winkelbereich von 0° bis 45° entsprechend bezogen auf die vorstehend genannten Achsen.

Weiterhin ist die erste Messöffnung derart am äußeren Umfang des Strömungskanals angeordnet, dass durch den Strömungskanal strömendes Atemgas Gas aus dieser mitreißt und so ähnlich dem Venturi-Prinzip bzw. einer Strahlpumpe eine lokalen Unterdruck-Komponente am ersten Messausgang erzeugt, der in Relation zur Strömungsgeschwindigkeit des Atemgases steht. Die zweite Messöffnung ist derart am äußeren Umfang des Strömungskanals angeordnet, dass das durch den Strömungskanal strömende Atemgas in diese hineindrückt und somit eine von der Strömungsgeschwindigkeit abhängenden, lokale Überdruck-Komponente am zweiten Messausgang erzeugt. Durch die Abhängigkeit von der Strömungsgeschwindigkeit sind die vorstehend benannte Unter- und Überdruck-Komponente als dynamische Druck-Komponenten definiert.

Die lokale Überlagerung von statischem und dynamischem Druck führt zu einer Veränderung des tatsächlich an den Abgriffen messbaren Differenzdrucks. Der messbare Druck nimmt auf der Seite des höheren statischen Drucks ab und auf der Seite des niedrigeren statischen Drucks zu.

Der messbare Druck ist die Summe des statischen und des Staudrucks am jeweiligen Messpunkt:

$$p_{total} = p_{stau} + p_{stat}$$

Aufgrund der unterschiedlichen lokalen Überlagerung von statischem und dynamischem Druck, ist der messbare Differenzdruck kleiner als die eigentliche Differenz der statischen Drücke an den beiden Messausgängen:

$$p_{stat1} + p_{stat2} > p_{total1} + p_{total2}$$

Der messbare Differenzdruck ist mit zunehmender Strömungsgeschwindigkeit stärker reduziert, wodurch die Kennlinie abflacht. Kleine Strömungen erzeugen nur einen kleinen Effekt, größere Strömungen einen entsprechend größeren Effekt.

Bei bekanntem Querschnitt des Strömungskanals kann aus der Differenz der sich jeweils aus statischer und dynamischer Druck-Komponente zusammensetzenden Gesamtdrücke an den Messausgängen auf den Volumenstrom geschlossen werden und dieser entsprechend der Erfordernisse geregelt werden.

Der Differenzdruck zeigt aufgrund der jeweiligen Überlagerung von dynamischem und statischem Druck bei einer erfindungsgemäß ausgebildeten Differenzdruck-Messstrecke eine, im Vergleich zu einer gewöhnlichen Differenzdruck-Messstrecke mit einer einfachen Blende oder einem einfachen Widerstand, im Hinblick auf die Auflösbarkeit bei geringen Volumenströmen verbesserte Kennlinie, die die Kennlinie des Systems, d.h. die Kombination der Kennlinien von Messstrecke und Drucksensor(en), einer linearen Kennlinie annähert.

Erfindungsgemäß sind Systemkennlinien zwischen einer linearen Kennlinie entsprechend einem Verlauf $a \cdot x^1 + b$ und einer Kennlinie mit einem Verlauf entsprechend $a \cdot x^{1,5} + b$ realisiert. In einer besonders vorteilhaften Ausführungsform ist eine Kennlinie mit einem Verlauf zwischen $a \cdot x^1 + b$ und etwa $a \cdot x^{1,3} + b$ realisierbar. Je nach eingesetztem Drucksensor kann der Koeffizient b ungefähr gleich 0 sein.

Die erfindungsgemäße Verbesserung der Übertragungskennlinie der Differenzdruck-Messvorrichtung ist somit durch eine Anpassung der Volumenstrom-Differenzdruck-Kennlinie der Differenzdruck-Messstrecke realisiert.

Das offenbarte Verfahren zur Messung des Differenzdrucks in einer Vorrichtung zur Beatmung verwendete die hier beschriebene Vorrichtung.

Weiterhin ist das offenbarte Verfahren zur Messung des Differenzdrucks in einer Vorrichtung zur Beatmung in einer vorteilhaften Ausführungsform dadurch gekennzeichnet, dass eine Bestimmung des Volumenstromes des Atemgases mithilfe einer Differenzdruck-Messvorrichtung vorgenommen wird, die eine Differenzdruck-Messstrecke aufweist, in der ein vom Volumenstrom des Atemgases abhängiger Differenzdruck erzeugt wird, und die einen Differenzdrucksensor aufweist, der den Differenzdruck erfasst und in eine elektronische Größe überführt, und dass der vorliegende Volumenstrom des Atemgases mithilfe eines Mikrocontrollers aus der von dem Differenzdrucksensor erzeugten elektronischen Größe bestimmt wird.

Darüber hinaus ist das offenbarte Verfahren zur Messung des Differenzdrucks in einer Vorrichtung zur Beatmung in einer besonders vorteilhaften Ausführungsform, dadurch gekennzeichnet, dass die zur Bestimmung des Volumenstromes des Atemgases verwendete Differenzdruck-Messstrecke Messstutzen aufweist, die derart an den Strömungskanal angeflanscht sind, dass die Ausrichtung der Messleitungen zumindest im Bereich der Messöffnungen eine wesentliche tangential zu äußeren Begrenzungsfläche des Strömungskanals und/oder der Hauptströmungsrichtung des Atemgases im Strömungskanal stehende Komponente aufweisen.

Verschiedene Ausführungsbeispiele und Ausgestaltungen der Erfindung sind in den nachfolgenden Figuren abgebildet. Es zeigen

Figur 1: Ein erfindungsgemäßes Beatmungsgerät in einer perspektivischen Darstellung,

Figur 2: ein Blockschaltbild einer erfindungsgemäßen Differenzdruck-Messvorrichtung eines Beatmungsgerätes,

Figur 3: in einer perspektivischen Darstellung eine erfindungsgemäß ausgebildete Differenzdruck-Messstrecke integriert in einen Adapter,

Figur 4: eine Draufsicht auf einen Schnitt durch den Adapter im Bereich der Differenzdruck-Messstrecke und

Figur 5: einen typischen Kennlinienverlauf für eine Volumenstrommessung.

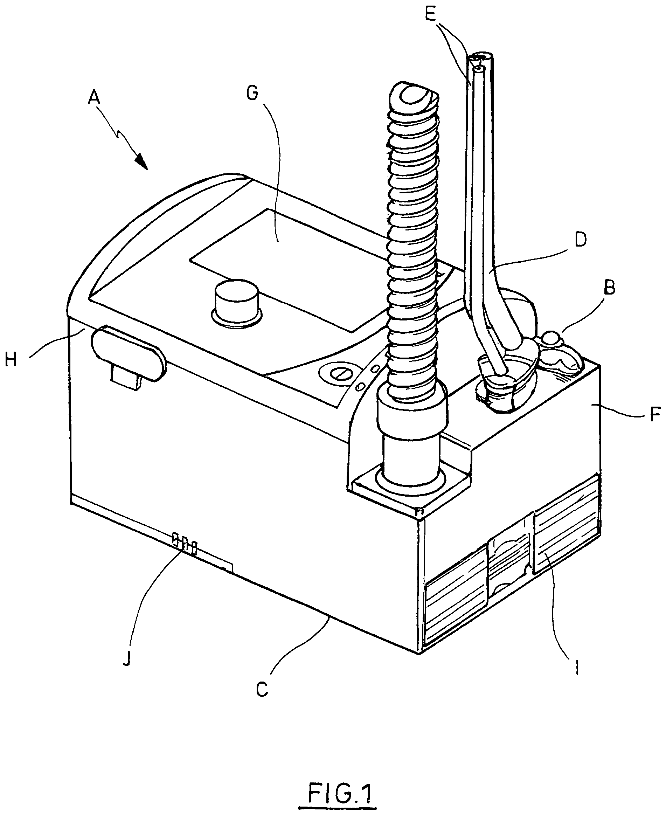

Figur 1 zeigt eine perspektivische Darstellung einer erfindungsgemäßen Vorrichtung zur Beatmung (A), aufweisend einen Druckanschluss (B) für einen Anschluss an eine Sauerstoffquelle, einen Anschluss für einen Beatmungsschlauch (C), über den das Atemgas zu einer Beatmungsmaske leitbar ist, und einen kombinierten Anschluss für einen Sauerstoffschlauch (D) und ein Messschlauchsystem (E), ausgebildet als ein Druckmessschlauch und ein PEEP-Steuerschlauch. Weiterhin weist die Vorrichtung zur Beatmung (A) einen Zubehöranschluss (F), ein Display (G), ein als ein Drehknopf ausgebildetes Bedienelement (H) und einen Lufteinlass (I) sowie einen Luftauslass (J) auf.

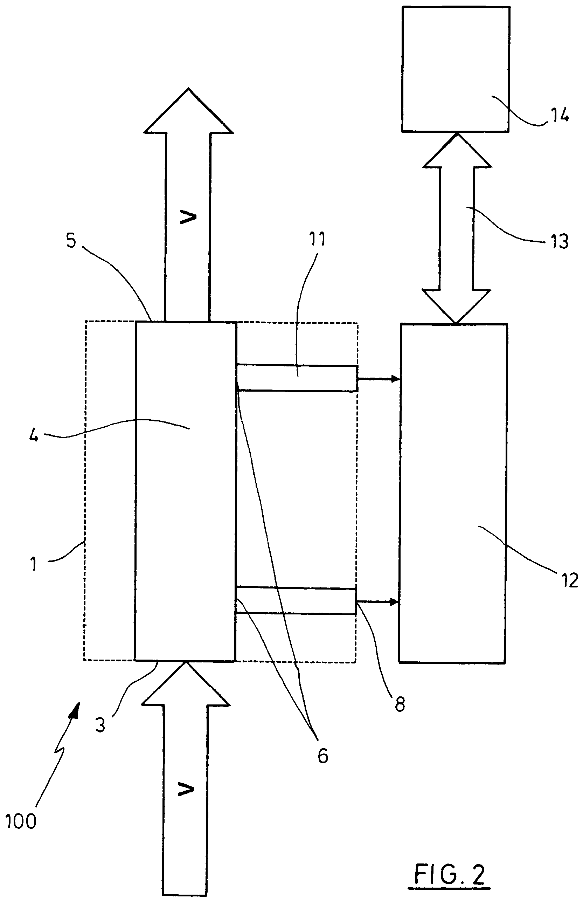

In Figur 2 ist ein ausschnittsweises Blockschaltbild einer Ausführungsform einer Vorrichtung zur Beatmung (A) dargestellt. Der gezeigte Ausschnitt betrifft die Differenzdruck-Messvorrichtung (100) der Vorrichtung zur Beatmung (A). Ein Atemgas strömt mit einem Volumenstrom (V) über eine Einströmöffnung (3) in eine Differenzdruck-Messstrecke (1), die einen Strömungskanal (4) aufweist, und am Ende des Strömungskanals (4) über eine Ausströmöffnung (5) aus diesem heraus. In der Differenzdruck-Messstrecke (1) erzeugt der Volumenstrom (V) lokale Drücke, die durch zwei Messöffnungen (6) über jeweils eine Messleitung (11) an einem zugeordneten Messausgang (8) anliegen. Mit den Messausgängen (8) verbunden ist ein Differenzdrucksensor (12), der die Drücke erfasst und in dem dargestellten Ausführungsbeispiel zudem digitalisiert. Die Druckmessdaten sind mithilfe eines Mikrocontrollers (14) über eine Schnittstelle (13), die als eine I2C-Schnittstelle realisiert ist, aus dem Differenzdrucksensor (12) auslesbar. Anhand der Systemparameter ist aus den digitalen Druckmessdaten mithilfe des Mikrocontrollers (14) der Volumenstrom (V) des Atemgases bestimmbar und für eine Regelung nutzbar.

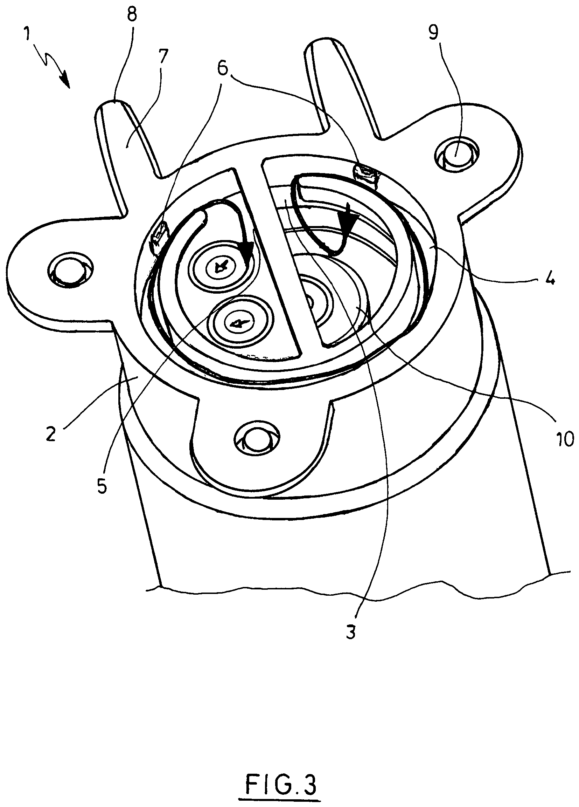

Figur 3 zeigt eine perspektivische Darstellung eines Schnittes der Erfindung einer Differenzdruck-Messstrecke (1), integriert in einen Adapter (2). Die Differenzdruck-Messstrecke (1) weist eine kreissegmentförmige Einströmöffnung (3) auf, durch die ein Atemgas mit einem durch die Vorrichtung zur Beatmung (A) geregelten Druck und/oder Volumenstrom (V) in die Differenzdruck-Messstrecke (1) einströmt. Von der Einströmöffnung (3) weg wird das Atemgas durch einen Strömungskanal (4) geleitet, der ringsegmentartig ausgebildet ist und einen rechteckigen Querschnitt aufweist. An seiner Oberseite ist der Strömungskanal (4) durch einen nicht dargestellten Deckel begrenzt und abgedichtet. An seinem anderen Ende weist der Strömungskanal (4) eine Ausströmöffnung (5) auf, durch die das Atemgas aus dem Strömungskanal (4) entweicht.

Es ist insbesondere an einen rechteckigen, einen quadratischen oder einen kreisförmigen Querschnitt des Strömungskanals (4) gedacht, da die Eigenschaften der Strömung für derart ausgebildete Kanäle bekannt sind und der Querschnitt einfach zu berechnen ist. Jedoch sind auch andere Querschnitte für einen Strömungskanal (4) einer Differenzdruck-Messstrecke (1) einer erfindungsgemäßen Vorrichtung zur Beatmung (A) denkbar.

An der radial außenliegenden Begrenzungsfläche des Strömungskanals (4) sind zwei Messöffnungen (6) angeordnet. An die Messöffnungen (6) schließen Druckmessstutzen (7) an, die mit einer wesentlichen tangentialen Richtungskomponente zur lokalen Hauptströmungsrichtung des Atemgases angeordnet sind. Am anderen Ende weisen die Druckmessstutzen (7) jeweils einen Messausgang (8) auf, an den eine Druckmesseinheit koppelbar ist. An dem die Differenzdruck-Messstrecke (1) integrierenden Adapter (2) sind radial außenliegend drei Befestigungsvorrichtungen (9) angeordnet, die als Schraubgewinde ausgebildet sind und für eine sichere Befestigung des Adapters (2) innerhalb der Vorrichtung zur Beatmung (A) dienen.

Weiterhin ist im Bereich der Einströmöffnung (3) der Differenzdruck-Messstrecke (1) ein gefedertes Überdruckventil (10) angeordnet, das bei einem Überdruck in der Atemgasleitung öffnet und den Druck reduziert.

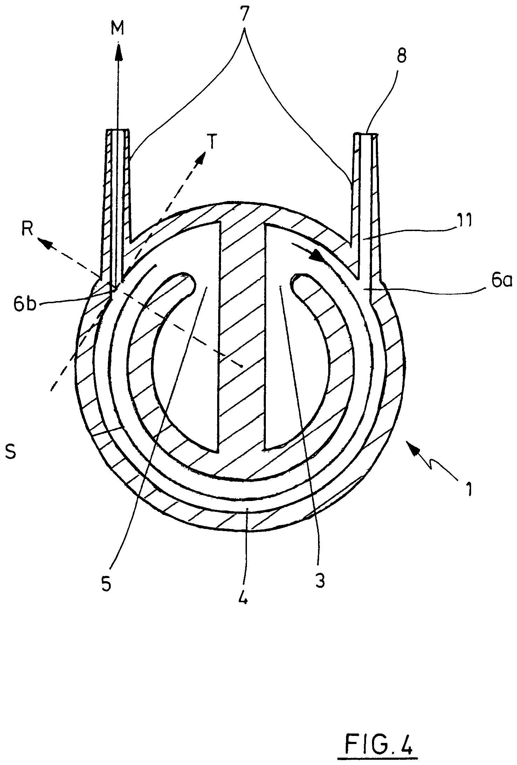

In Figur 4 ist ein Schnitt der Differenzdruck-Messstrecke (1) dargestellt. Der Strömungskanal (4) wird radial innen- und außenseitig durch das Material des Adapters (2) in einer ringsegmentartigen Kontur begrenzt. Durch die ringartige Ausbildung des Strömungskanals (4) ist eine besonders kompakte Bauform der Differenzdruck-Messstrecke (1) realisiert. Zudem ist die dargestellte erfindungsgemäße Anordnung der Druckmessstutzen (7) bzw. der Messleitungen (11) innerhalb der Druckmessstutzen (7) mit einer wesentlichen zur lokalen Hauptströmungsrichtung (S) des Atemgases bzw. zur lokalen äußeren Begrenzungsfläche des Strömungskanals (4) im Bereich der Messöffnung (6) tangentialen Richtungskomponente realisiert.

Eine lokal (im Bereich der Messöffnung) tangential ausgerichtete Achse (T) spannt mit einer lokal radial ausgerichteten Achse (R) eine Ebene auf, in der die Ausrichtung (M) einer Messleitung (11) in einem Bereich von 0° bis 60° um die tangential ausgerichtete Achse (T) liegt.

Innerhalb des ringsegmentartig ausgebildeten Strömungskanals (4), ist die lokale Hauptströmungsrichtung (S) des Atemgases im Wesentlichen tangential zur Kreisform der radial außenliegenden Kanalbegrenzung ausgerichtet. Sind die Messleitungen (11) zumindest im Bereich der Messöffnungen (6) mit einer wesentlichen Richtungskomponente (M) in dieser tangentialen Richtung T angeordnet, beeinflusst dies erfindungsgemäß die Volumenstrom- Differenzdruck-Kennlinie der Differenzdruck-Messstrecke (1) derart, dass sich die Volumenstrom- Differenzdruck-Kennlinie der Differenzdruck-Messvorrichtung von der bekannten quadratischen Abhängigkeit der Kennlinie in Richtung einer linearen Kennlinie annähert.

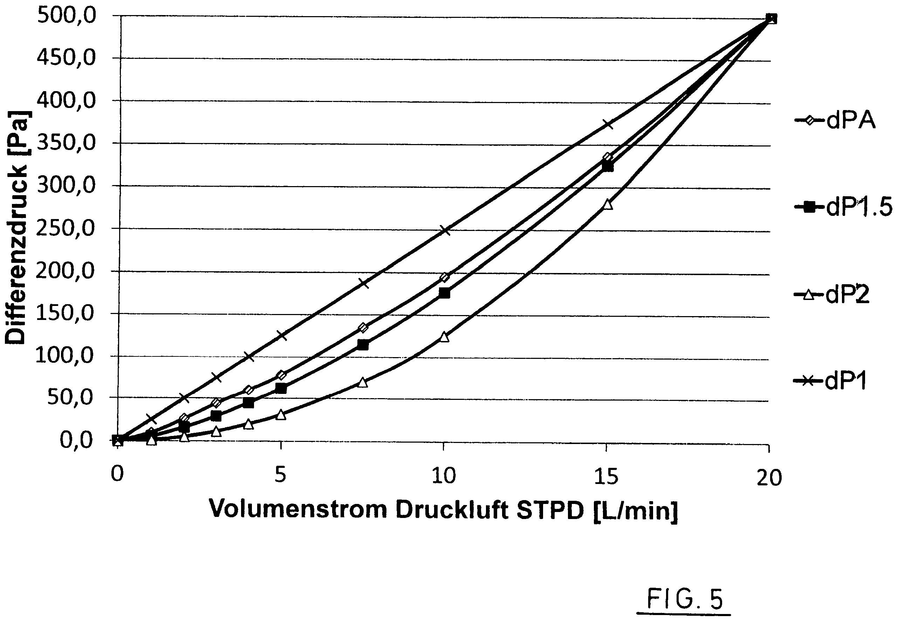

Figur 5 zeigt ein Diagramm mit verschiedenen Volumenstrom-Differenzdruck-Kennlinien, die jeweils die Systemkennlinie über Differenzdruck-Messstrecke und Drucksensoren darstellen. In dieser Darstellung ist ein angestrebter Volumenstrom-Messbereich von 20 L/min STPD (Standard Temperature and Pressure Dry) festgelegt und durch den eingesetzten Differenzdrucksensor ein maximaler Messbereich von 500 Pa gegeben.

Der Differenzdruck steigt für eine ideale Auflösbarkeit linear an (dP1). In diesem Fall sind die Differenzdrücke bereits im unteren Volumenstrombereich hinreichend hoch, um gut auflösbar zu sein.

Bei einer gewöhnlichen Differenzdruck-Messstrecke (einfache Blende oder einfacher Widerstand) zeigt sich typischerweise ein Verhalten Differenzdruck proportional zum Quadrat des Volumenstroms (dP2). In diesem Fall sind die Differenzdruck-Messwerte im unteren Volumenstrombereich sehr klein.

Die hier beschriebene erfindungsgemäße Konstruktion ermöglicht mit einem einfachen Aufbau eine Kennlinie (Differenzdruck über Volumenstrom), bei dem der Differenzdruck etwa proportional zur Potenz 1,5 des Volumenstroms ist (dP1.5).

Eine Druckmessung mit einer erfindungsgemäßen Differenzdruck-Messvorrichtung zeigte eine Volumenstrom-Differenzdruck-Kennlinie proportional zur Potenz 1,3 (dPA).

**Patentansprüche**

1. Vorrichtung zur Beatmung (A), aufweisend mindestens eine Differenzdruck-Messstrecke (1) und einen Differenzdrucksensor (12), wobei die Differenzdruck-Messstrecke (1) einen Strömungskanal (4) mit mindestens zwei zwischen einer Einströmöffnung (3) und einer Ausströmöffnung (5) des Strömungskanals (4) angeordneten Messöffnungen (6) aufweist, an die sich in Messstutzen (7) angeordnete Messleitungen (11) anschließen, wobei ein Atemgas von der Einströmöffnung (3) in einer Hauptströmungsrichtung (S) durch den Strömungskanal (4) zur Ausströmöffnung (5) geleitet wird und wobei durch die Differenzdruck-Messstrecke (1) in Verbindung mit dem Differenzdrucksensor (12) eine Volumenstrom-Differenzdruck-Kennlinie realisiert ist, die zwischen einem linearen $a \cdot x^{1,0} + b$ und einem durch $a \cdot x^{1,5} + b$ beschreibbaren Verlauf liegt, wobei je nach eingesetztem Drucksensor der Koeffizient b ungefähr gleich 0 ist,
wobei der Strömungskanal (4) in der Hauptströmungsrichtung (S) des Atemgases durch den Strömungskanal (4) zumindest teilweise ringförmig oder als ein Ringsegment ausgebildet ist, dass die Messstutzen (7) derart an den Strömungskanal (4) angeflanscht sind, dass die Ausrichtung (M) der Messleitungen (11) zumindest im Bereich der Messöffnungen (6) eine wesentliche tangential zu äußeren Begrenzungsfläche des Strömungskanals (4) und/oder der Hauptströmungsrichtung (S) des Atemgases im Strömungskanal (4) stehende Komponente aufweisen und dass die Ausrichtung (M) einer Messleitung (11) zumindest im Bereich einer Messöffnung (6) in einem Winkelbereich von 0° bis 60°, gemessen von der, durch die lokale Tangente definierten Achse (T), in Richtung einer dazu orthogonalen, sich in radialer Richtung erstreckenden Achse (R), liegt.

2. Vorrichtung nach mindestens einem der vorstehenden Ansprüche, **dadurch gekennzeichnet dass** bei einer definierten Strömungsrichtung eines Atemgases durch den Strömungskanal (4) der Differenzdruck-Messstrecke (1) im Bereich einer ersten Messöffnung (6a) ein statischer Druck und ein dynamischer, von dem Volumenstrom (V) abhängiger Unterdruck und im Bereich einer zweiten Messöffnung (6b) ein statischer Druck und ein dynamischer, vom Volumenstrom (V) abhängiger Überdruck vorliegt.

3. Vorrichtung nach mindestens einem der vorstehenden Ansprüche, **dadurch gekennzeichnet, dass** die Ausrichtung (M) der Messleitung (11) im Bereich einer Messöffnung (6) für eine erste Messöffnung (6a) in etwa entgegen der Hauptströmungsrichtung (S) und für eine zweite Messöffnung (6b) in etwa in Richtung der Hauptströmungsrichtung (S) realisiert ist.

4. Vorrichtung nach mindestens einem der vorstehenden Ansprüche, **dadurch gekennzeichnet, dass** diese als ein Notfallbeatmungsgerät ausgebildet ist.

5. Vorrichtung nach Anspruch 8, **dadurch gekennzeichnet, dass** diese als ein mobiles Notfallbeatmungsgerät ausgebildet ist.

## Claims

1. Ventilator apparatus (A), comprising at least one differential pressure measurement path (1) and a differential pressure sensor (12), wherein the differential pressure measurement path (1) has a flow channel (4) with at least two measurement openings (6) which are arranged between an inflow opening (3) and an outflow opening (5) of the flow channel (4) and which are adjoined by measurement lines (11) arranged in measurement nozzles (7), wherein a respiratory gas is guided from the inflow opening (3) to the outflow opening (5) through the flow channel (4) in a principal flow direction (S) and wherein a volumetric flow rate/differential pressure characteristic is realized by the differential pressure measurement path (1) in conjunction with the differential pressure sensor (12), said characteristic lying between a linear curve $a \cdot X^{1.0} + b$ and a curve describable by $a \cdot X^{1.5} + b$, wherein the coefficient b is approximately equal to 0 depending on the pressure sensor employed, wherein, in the principal flow direction (S) of the respiratory gas through the flow channel (4), the flow channel (4) has an at least partly ring-shaped embodiment or an embodiment as a ring segment, that the measurement nozzles (7) are flanged onto the flow channel (4) in such a way that the alignment (M) of the measurement lines (11), at least in the region of the measurement openings (6), has an essential component that is tangential to the outer delimiting face of the flow channel (4) and/or to the principal flow direction (S) of the respiratory gas in the flow channel (4) and that the alignment (M) of a measurement line (11), at least in the region of a measurement opening (6), lies in an angular range of 0° to 60°, as measured from the axis (T) defined by the local tangent in the direction of an axis (R) which is orthogonal thereto and extends in the radial direction.

2. Apparatus according to at least one of the preceding claims, **characterized in that**, given a defined flow direction of a respiratory gas through the flow channel (4) of the differential pressure measurement path (1), a static pressure and a dynamic, volume flow (V) dependent negative pressure are present in the region of a first measurement opening (6a) and a static pressure and a dynamic, volume flow (V) dependent positive pressure are present in the region of a second measurement opening (6b).

3. Apparatus according to at least one of the preceding claims, **characterized in that** the alignment (M) of the measurement line (11), in the region of a measurement opening (6), is realized approximately counter to the principal flow direction (S) for a first measurement opening (6a) and approximately in the direction of the principal flow direction (S) for a second measurement opening (6b).

4. Apparatus according to at least one of the preceding claims, **characterized in that** the latter is embodied as an emergency ventilator.

5. Apparatus according to Claim 8, **characterized in that** the latter is embodied as a mobile emergency ventilator.

## Revendications

1. Dispositif d'assistance respiratoire (A), présentant au moins une section de mesure de pression différentielle (1) et un capteur de pression différentielle (12), la section de mesure de pression différentielle (1) présentant un canal d'écoulement (4) doté d'au moins deux orifices de mesure (6) disposés entre un orifice d'entrée (3) et un orifice de sortie (5) du canal d'écoulement (4), prolongés par des lignes de mesure (11) disposées dans des raccords de mesure (7), un gaz respiratoire étant conduit de l'orifice d'entrée (3) dans une direction d'écoulement principale (S) à travers le canal d'écoulement (4) jusqu'à l'orifice de sortie (5), et la section de mesure de pression différentielle (1) en conjonction avec le capteur de pression différentielle (12) réalisant une courbe caractéristique de pression différentielle de débit volumique qui se situe entre une allure linéaire $a \cdot x^{1.0} + b$ et une allure pouvant être décrite par $a \cdot x^{1.5} + b$, le coefficient b étant approximativement égal à 0 en fonction du capteur de pression mis en œuvre, le canal d'écoulement (4) étant réalisé dans la direction d'écoulement principale (S) du gaz respiratoire à travers le canal d'écoulement (4) au moins partiellement en forme d'anneau ou de segment d'anneau, que les raccords de mesure (7) sont bridés sur le canal d'écoulement (4) de telle sorte que l'orientation (M) des lignes de mesure (11) présentent au moins au niveau des orifices de mesure (6) un composant substantiellement tangentiel à la surface de délimitation extérieure du canal d'écoulement (4) et/ou à la direction d'écoulement principale (S) du gaz respiratoire dans le canal d'écoulement (4), et que l'orientation (M) d'une ligne de mesure (11) se trouve au moins au niveau d'un orifice de mesure (6) dans une plage d'angle de 0° à 60°, mesurée à partir de l'axe (T) défini par la tangente locale, en direction d'un axe (R) orthogonal à celui-ci et s'étendant dans la direction radiale.

2. Dispositif selon au moins l'une des revendications précédentes, **caractérisé en ce que** pour une direction d'écoulement définie d'un gaz respiratoire à travers le canal d'écoulement (4) de la section de mesure de pression différentielle (1) au niveau d'un premier orifice de mesure (6a), une pression statique et une dépression dynamique dépendant du débit volumique (V) sont présentes, et au niveau d'un deuxième orifice de mesure (6b), une pression statique et une dépression dynamique dépendant du débit volumique (V) sont présentes.

3. Dispositif selon au moins l'une des revendications précédentes, **caractérisé en ce que** l'orientation (M) de la ligne de mesure (11) au niveau d'un orifice de mesure (6) est réalisée pour un premier orifice de mesure (6a) approximativement à l'opposé de la direction d'écoulement principale (S), et pour un deuxième orifice de mesure (6b) approximativement en direction de la direction d'écoulement principale (S).

4. Dispositif selon au moins l'une des revendications précédentes, **caractérisé en ce qu'**il est réalisé sous la forme d'un respirateur d'urgence.

5. Dispositif selon la revendication 8, **caractérisé en ce qu'**il est réalisé sous la forme d'un respirateur d'urgence mobile.

FIG.1

FIG. 2

FIG.3

FIG.4

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0552916 A1
- DE 10035938
- DE 102014119146
- EP 0331772 A
- US 5379650 A
- US 4403514 A
- US 2008092898 A

**In der Beschreibung aufgeführte Nicht-Patentliteratur**

- **J P KIRKNESS.** *Pitot-tube flowmeter for quantification of airflow during sleep*